# EUROPEAN PATENT SPECIFICATION

(11) **EP 3 333 583 B1**
(45) Date of publication and mention of the grant of the patent: **29.12.2021**
(21) Application number: 17204296.2
(22) Date of filing: 29.11.2017
(51) Int. Cl.: G01R 33/48, G01R 33/54, G01R 33/56, G01R 33/58, A61B 5/055, A61N 5/10

(54) **METHOD FOR IDENTIFYING AN ORGAN STRUCTURE OF AN INVESTIGATED OBJECT IN MAGNETIC RESONANCE IMAGE DATA**
VERFAHREN ZUR IDENTIFIZIERUNG EINER ORGANSTRUKTUR EINES UNTERSUCHTEN OBJEKTS IN MAGNETRESONANZBILDDATEN
PROCÉDÉ D'IDENTIFICATION D'UNE STRUCTURE D'ORGANE D'UN OBJET À EXAMINER DANS DES DONNÉES D'IMAGE PAR RÉSONANCE MAGNÉTIQUE

(30) Priority: 06.12.2016 US 201615370062
(43) Date of publication of application: 13.06.2018
(73) Proprietor: Siemens Healthcare GmbH, 91052 Erlangen (DE)
(72) Inventor: Amies, Christopher Jude, Sykesville, 21784 (US); Hengerer, Arne, 91096 Möhrendorf (DE); Schneider, Rainer, 91054 Erlangen (DE)

(56) References cited:
- US-A1- 2015 343 237
- KONAR A.S. ET AL: "Region of interest compressive sensing (ROICS)", PROCEEDINGS OF THE INTERNATIONAL SOCIETY FOR MAGNETIC RESONANCE IN MEDICINE, 21ST ANNUAL MEETING AND EXHIBITION, SALT LAKE CITY, UTAH, USA, 20-26 APRIL 2013, vol. 21, 6 April 2013 (2013-04-06), page 3801, XP040631397,
- KIM Y.-C. ET AL: "Edge detection using sub-sampled k-space data: application to upper airway MRI", PROCEEDINGS OF THE INTERNATIONAL SOCIETY FOR MAGNETIC RESONANCE IN MEDICINE, 15TH ANNUAL MEETING AND EXHIBITION, BERLIN, GERMANY, 19-25 MAY 2007, vol. 15, 5 May 2007 (2007-05-05), page 3458, XP040602637,
- KUSTNER T ET AL: "MR Image Reconstruction Using a Combination of Compressed Sensing and Partial Fourier Acquisition: ESPReSSo", IEEE TRANSACTIONS ON MEDICAL IMAGING, IEEE SERVICE CENTER, PISCATAWAY, NJ, US, vol. 35, no. 11, 1 November 2016 (2016-11-01), pages 2447-2458, XP011626894, ISSN: 0278-0062, DOI: 10.1109/TMI.2016.2577642 [retrieved on 2016-10-27]

## Description

The invention relates to a method for identifying an organ structure of an investigated object in magnetic resonance image data, a magnetic resonance machine, and a computer program product.

In a magnetic resonance machine, also referred to as a magnetic resonance tomography system, the body to be investigated, of an investigated object, for example of a patient, a healthy volunteer, an animal or a phantom, is usually exposed with the aid of a main magnet to a relatively high main magnet field, of 1.5 or 3 or 7 tesla for example. Additionally, gradient switching processes are run through with the aid of gradient coil unit. By means of a radio-frequency antenna unit, radio-frequency pulses, for example excitation pulses, are then emitted by way of suitable antenna facilities, which results in the nuclear spins of certain atoms excited in a resonant manner by these radio-frequency pulses being tilted over by a defined flip angle as compared with the magnetic field lines of the main magnet field. During relaxation of the nuclear spins, radio-frequency signals, so-called magnetic resonance signals, are emitted which are received by way of suitable radio-frequency antennas and then undergo further processing. Lastly, the desired image data can be reconstructed from the raw data acquired in this way.

For a certain measurement, therefore, a certain magnetic resonance sequence, also referred to as a pulse sequence, must be emitted, which sequence consists of a succession of radio-frequency pulses, for example excitation pulses and refocusing pulses, and also gradient switching processes to be emitted appropriately coordinated with this in various gradient axes along various spatial directions. Timed appropriately with this, read-out windows are set which specify the time periods in which the induced magnetic resonance signals are captured.

Furthermore, various options are known for identifying an organ structure in magnetic resonance image data which is reconstructed from magnetic resonance measurement data acquired by way of a magnetic resonance machine. In this regard, the organ structure can be an entire bodily organ of the investigated object or a part of a bodily organ of the investigated object. In addition to manual segmentation, various semi-automatic or automatic segmentation methods are known for identifying the organ structure in the magnetic resonance image data. Irrespective of the method used for identifying the organ structure, the outcome or the quality of the identification of the organ structure is typically dependent on an image quality of the magnetic resonance image data. In this regard, a high level of image quality can typically result in a robust identification of the organ structure.

The identification of an organ structure in magnetic resonance image data can be needed for planning a radiation treatment of the investigated object or patient for example. During a radiation treatment, a target tissue of a patient such as a tumor is irradiated with ionizing radiation. In this regard, external radiotherapy is well-known, comprising radiation treatment of the body of the patient from outside the body. Internal radiotherapy, also referred to as brachytherapy, is likewise well-known.

Given the magnetic resonance image data, it is firstly possible to define the target volume of the radiation treatment and secondly to localize surrounding tissue to be spared - for example neural tissue. Being the target organ, the identified organ structure can serve in this way as the basis for defining the target volume of the radiation treatment. Also, being the risk organ, the identified organ structure can serve as the basis for defining the tissue to be spared. The identified organ structure can also be included in a calculation of an electron density map, which serves as the basis of a dose calculation for planning the radiation treatment.

Alongside computer tomography image data, magnetic resonance image data is in fact being employed increasingly frequently for the planning of radiation treatment since magnetic resonance image data has an improved soft tissue contrast and in this manner enables improved identification of target organs and/or risk organs. Furthermore, a development of the last few years under the banner of exclusively magnetic resonance-based radiation treatment planning ("MR-only RT Planning", MRORTP), provides for eliminating computer tomography image data from the planning process for suitable clinical applications. In this manner, radiation treatment planning should be effected exclusively on the basis of magnetic resonance image data acquired from the patient. In this way for example, the quantity of necessary patient pictures (just magnetic resonance pictures in place of computer tomography pictures and magnetic resonance pictures) can be reduced and/or possible registration errors between the CT image data and magnetic resonance image data avoided.

US 2016/0059041 A1 describes an MR-based method to monitor a radiation therapy of a patient. US 2015/0343237 A1 describes using MR-data in order to determine a target region for radiotherapy. US 2016/0086330 A1 describes determining periodic movement of a tumor on the basis of 4D MRI images in order to compile a more efficient treatment plan.

The paper of Konar et al. "Region of interest compressive sensing (ROICS)" PROCEEDINGS OF THE INTERNATIONAL SOCIETY FOR MAGNETIC RESONANCE IN MEDICINE, 21ST ANNUAL MEETING AND EXHIBITION, SALT LAKE CITY, UTAH, USA, 20-26 APRIL 2013, vol. 21, 6 April 2013 (2013-04-06), page 3801, describes a MRI compressed sensing reconstruction with additional sparsity and limited to a certain region of interest. The paper of Kim et al. "Edge detection using sub-sampled k-space data: application to upper airway MRI", PROCEEDINGS OF THE INTERNATIONAL SOCIETY FOR MAGNETIC RESONANCE IN MEDICINE, 15TH ANNUAL MEETING AND EXHIBITION, BERLIN, GERMANY, 19-25 MAY 2007, vol. 15, 5 May 2007 (2007-05-05), page 3458, describes a method to detect tissue borders in magnetic resonance images of the upper airway of a subject while during the k-space acquisition only 50 percent of the k-space has been sampled in spiral and spiral-ring trajectories.

The article by Kustner et al. "MR Image Reconstruction Using a Combination of Compressed Sensing and Partial Fourier Acquisition: ESPReSSo", IEEE TRANSACTIONS ON MEDICAL IMAGING, vol. 35, no. 11, 1 November 2016, pages 2447-2458, describes a method for applications which request well-defined tissue boundaries (i.e. high edge delineation) in high spatial/ temporal resolved images of compressed sensing accelerated scans, and recognizes the advantage of a denser sampling of the high-frequency components of k-space compared to other compressed sensing methods, while however sampling the low frequency components of k-space with the highest density.

The independent method claim is drafted in a two-part form with respect to this paper The object underlying the invention is to enable an improved identification of an organ structure of an investigated object in magnetic resonance image data. The object is achieved by the features of the independent claims. Advantageous embodiments are described in the sub claims.

The inventive method for identifying an organ structure of an investigated object in magnetic resonance image data according to independent claim 1 comprises the following method steps:
- acquisition of magnetic resonance measurement data for the organ structure of the investigated object by way of a magnetic resonance machine using a magnetic resonance sequence which specifies a sampling scheme of a k-space,
- reconstruction of magnetic resonance image data from the magnetic resonance measurement data, and
- identification of the organ structure in the magnetic resonance image data, wherein the identification of the organ structure in the magnetic resonance image data comprises a segmentation of the organ structure by way of a segmentation algorithm and/or a contouring of the organ structure,
   wherein the sampling scheme of the k-space supports the subsequent identification of the organ structure in the magnetic resonance image data reconstructed from the magnetic resonance measurement data,
   characterized in that the sampling scheme of the k-space supports the subsequent identification of the organ structure in the manner that the sampling scheme of the k-space provides for a higher sampling density in an outer region of the k-space than in a central region of the k-space,
   wherein the higher sampling density in the outer region than in the central region results in an emphasizing of an outer contour of the organ structure in the reconstructed magnetic resonance image data,
   wherein the identification of the organ structure is effected using the emphasized outer contour of the organ structure in the magnetic resonance image data.

The magnetic resonance measurement data is typically captured by applying phase coding gradients and frequency coding gradients and recording the resulting magnetic resonance signals by way of a radio-frequency coil. The magnetic resonance signals read out during capture of the magnetic resonance measurement data are typically deposited in a k-space. In this regard, the k-space can be seen as a spatial frequency domain. During acquisition of the magnetic resonance measurement data, the k-space is usually filled by way of a deterministic sampling scheme which is specified by the magnetic resonance frequency. In this way, the magnetic resonance measurement data is typically just the raw data containing the magnetic resonance signals recorded in the spatial frequency domain. The magnetic resonance measurement data is therefore not typically directly available to a competent person for the purpose of diagnosis.

Instead, magnetic resonance image data is reconstructed from the magnetic resonance measurement data, which image data can be presented on a display unit and/or can be made available to a competent person for establishing a diagnosis. In this regard, reconstruction of the magnetic resonance image data from the magnetic resonance measurement data comprises in particular the generation of magnetic resonance images deposited in an image domain from the magnetic resonance signals deposited in the k-space. In this regard, the reconstruction of the magnetic resonance image data can be effected by way of a method familiar to a person skilled in the art, for example a Fourier transform.

Identification of the organ structure comprises in particular an automatic, semi-automatic or manual recognition of the organ structure in the magnetic resonance image data. The identification of the organ structure can comprise a determination of which part of the magnetic resonance image data is attributable to the organ structure. In this manner, the identification of the organ structure comprises in particular defining for each voxel in the magnetic resonance image data whether the voxel belongs to the organ structure or not. Automatic or semi-automatic identification of the organ structure can comprise a segmentation of the organ structure by way of a segmentation algorithm. In this regard, the segmentation algorithm can employ a known segmentation method, for example an active contours segmentation method (e.g. active snakes), a level-set segmentation method, a region growing segmentation method, or a statistical segmentation method (e.g. active shape models). In the case of semi-automatic segmentation, the user can initialize the segmentation, for example by setting a seed point and/or by setting at least one landmark. The user can also monitor and/or change the segmentation effected. Identification of the organ structure can also comprise a contouring of the organ structure, especially in the context of planning a radiation treatment of the investigated object. In this regard, the contouring can be effected with the support of automatic image processing methods for the magnetic resonance image data.

In particular, the identified organ structure is delivered, that is to say output on an output unit such as a display unit and/or stored in a database. Alternatively or additionally, the identified organ structure can be passed to a further processing unit which can carry out further processing of the magnetic resonance image data based on the identified organ structure. With regard to the case of planning a radiation treatment of the investigated object, the identified organ structure can be set as the target organ or risk organ for the radiation treatment planning.

The proposed procedure is based particularly on the approach of coordinating the sampling scheme of the k-space to the subsequent identification of the organ structure. In this way, acquisition of the magnetic resonance measurement data can be optimized such that the organ structure can be identified particularly simply or precisely in the magnetic resonance image data reconstructed from the magnetic resonance measurement data. The magnetic resonance measurement data is advantageously realized in such a manner that the organ structure can be segmented and/or contoured particularly simply or precisely in the magnetic resonance image data reconstructed from the magnetic resonance measurement data. Advantageously, the sampling scheme of the k-space can be coordinated specifically to the organ structure to be identified. A magnetic resonance sequence employed for the purpose of acquiring the magnetic resonance measurement data can therefore be of such a kind that it specifies a sampling scheme which generates particularly suitable magnetic resonance measurement data for the subsequent identification of the organ structure. Alternatively or additionally, parameters of such a kind can be set as settings for the magnetic resonance sequence that the sampling scheme generates particularly suitable magnetic resonance measurement data for the subsequent identification of the organ structure based on the parameters.

The sampling scheme can be set as a function of magnetic resonance preliminary measurement data acquired in a preliminary measurement effected prior to the acquisition of the magnetic resonance measurement data. The preliminary measurement can be realized for example in the form of a localizer measurement or an auto-align measurement. Advantageously, the magnetic resonance preliminary measurement data already comprises measurement data from the organ structure of the investigated object.

In a particularly advantageous manner, identification of the organ structure can comprise a recognition of an outer contour of the organ structure in the magnetic resonance image data. In this regard, the outer contour of the organ structure can represent an outer boundary of the organ structure with respect to the surrounding tissue. In particular, the outer contour of the organ structure can therefore also be designated as the outline or profile or outer limit or outer line of the organ structure. By recognizing the outer contour of the organ structure, it is therefore possible to define particularly simply which part of the magnetic resonance image data is attributable to the organ structure. Advantageously, the sampling scheme can therefore support identification of the organ structure in a manner such the sampling scheme supports recognition of the outer contour of the organ structure in the magnetic resonance image data. In this manner, the sampling scheme is advantageously realized in such a manner that the outer contour of the organ structure is presented in a emphasized manner or is easily recognizable in the magnetic resonance image data reconstructed from the magnetic resonance measurement data. In this manner, the sampling scheme can be designed specifically for measurement of the outer contour of the organ structure. In this manner, a direct visualization of the outer organ contour can be present in the magnetic resonance image data. This can then be used for identification of the organ structure. An internal structure of the organ structure can naturally be visible additionally in the magnetic resonance image data reconstructed from the magnetic resonance measurement data. Options for configuring the sampling scheme to simplify recognition of the outer contour of the organ structure are described in the embodiments.

The magnetic resonance image data acquired by way of the proposed procedure can be employed particularly advantageously for preparing the planning of a radiation treatment of the investigated object. In particular, an especially rapid and/or simple and/or precise identification of the organ structure in the magnetic resonance image data is possible. The identified organ structure can be used as the target organ or risk organ for planning the radiation treatment of the investigated object. The proposed procedure can therefore improve particularly advantageously the contouring of the organ structure which is needed for the subsequent planning of the radiation treatment. Automation of the contouring of the organ structure can also be supported advantageously in this way. In this manner, a user-independent or standardized contouring of the organ structure can be achieved.

Advantageously, the proposed procedure can also enable a particularly simple modeling of a movement of the organ structure if the magnetic resonance image data maps the organ structure in a time-resolved manner. In this manner, the sampling scheme can be optimized for tracking the outer contour of the organ structure in the magnetic resonance image data over the period of acquisition of the magnetic resonance measurement data. To this effect, a 4D cine magnetic resonance sequence can be employed for acquisition of the magnetic resonance measurement data for example. Where the outer contour of the organ structure can be identified in a time-resolved manner, movement correction of the magnetic resonance image data can be employed for example. Deployment in adaptive radiotherapy is also conceivable, particularly in the case of use in a hybrid magnetic resonance radiation treatment unit, where the magnetic resonance measurement data is acquired from the investigated object and the investigated object is treated with radiation in the same session. Optimization of the sampling scheme therefore allows improved tracking of the volume of the organ structure in the magnetic resonance image data, for example compared with the use of navigator techniques, to be enabled.

According to the inventive method the sampling scheme of the k-space supports the subsequent identification of the organ structure in the manner that the sampling scheme of the k-space provides for a higher sampling density in an outer region of the k-space than in a central region of the k-space.

In particular, the k-space is subdivided in to a central region and an outer region, wherein the outer region surrounds the central region. The central region of the k-space comprises in particular a center of the k-space. In this manner, magnetic resonance measurement data is deposited in the central region of the k-space, which data corresponds to lower spatial frequencies than the magnetic resonance measurement data deposited in the outer region of the k-space.

The fact that the sampling scheme of the k-space provides for a higher sampling density in the outer region of the k-space than in the central region of the k-space can mean that in the outer region, the sampling points are situated more densely than in the central region. As described more precisely in the next embodiment, it is also possible for no sampling at all to be effected in the central region of the k-space. Alternatively, it is also conceivable that the entire k-space is sampled uniformly and that exclusively or primarily the sampling points from the outer region of the k-space are used for reconstruction of the magnetic resonance image data.

The magnetic resonance measurement data deposited in the outer region of the k-space, which corresponds to the high spatial frequencies, is typically responsible for sharpness information or edge information in the reconstructed magnetic resonance image data. Consequently, an increased sampling of the k-space in the outer region can advantageously result in an emphasizing of the outer contours of the organ structure in the magnetic resonance image data reconstructed from the magnetic resonance measurement data. In this manner, the organ structure in the magnetic resonance image data acquired in this way can be identified particularly simply. On the other hand, a higher sampling density in the central region than in the outer region of the k-space is usually present in conventional undersampling methods.

An embodiment provides that the sampling scheme of the k-space provides for a sampling of the k-space exclusively in the outer region.

In this manner in particular, no sampling of the k-space is effected in the central region of the k-space. In particular, just the higher spatial frequencies in the k-space are sampled. In this manner, the outer contour of the organ structure in the magnetic resonance image data can be emphasized particularly clearly so that identification of the organ structure can be effected particularly simply. The boundary between the central region and the outer region in the k-space can be set in line with the spatial sharpness needed for identifying the organ structure.

An embodiment provides that the sampling scheme of the k-spaces provides for a spiral or radial sampling of the k-space with a higher sampling density in the outer region than in the central region.

It is also conceivable for the spiral or radial sampling of the k-space to be effected exclusively in the outer region. In this manner for example, one outer arc of a spiral can be sampled exclusively for acquisition of the magnetic resonance measurement data. In the present case, the spiral or radial sampling means that a time saving and/or reduction in movement artifacts can additionally be achieved particularly advantageously.

According to the inventive method the higher sampling density in the outer region than in the central region results in an emphasizing of an outer contour of the organ structure in the reconstructed magnetic resonance image data, wherein the identification of the organ structure is effected using the emphasized outer contour of the organ structure in the magnetic resonance image data.

The emphasized outer contour of the organ structure can markedly simplify segmentation or contouring of the organ structure. In this manner, the organ structure can thus be identified particularly advantageously in the magnetic resonance image data.

An embodiment provides that the sampling scheme of the k-space is determined based on training magnetic resonance image data, wherein the training magnetic resonance image data has been acquired from at least one subject other than the investigated object, and the organ structure of the at least one subject is already present and identified in the training magnetic resonance image data.

The training magnetic resonance image data can have been captured similarly to the magnetic resonance image data and stored in an atlas. The training magnetic resonance image data is advantageously acquired from a plurality of subjects other than the investigated object so that a larger population basis underlies the training magnetic resonance image data. In particular, a manual identification such as contouring of the organ structure of the at least one subject, possibly with the aid of automatic image processing techniques, is effected. Naturally, the same organ structure that is to be identified in the concrete case is in particular already present and identified in the training magnetic resonance image data. Furthermore, parameters of the sampling scheme used in each case for acquisition of the training magnetic resonance image data can be deposited with respect to the training magnetic resonance image data.

The organ structure already present and identified in the training magnetic resonance image data can represent a valuable basis for determination of the sampling scheme. In this manner, the sampling scheme can be tailored particularly suitably to identification of the organ structure. An advantageous procedure in this regard is described in the next embodiment.

An embodiment provides that the determining of the sampling scheme of the k-space based on the training magnetic resonance image data comprises a calculation of a sampling mask based on an outer contour of the organ structure of the at least one subject identified in the training magnetic resonance image data, wherein the sampling scheme of the k-space provides for a sampling of the k-space according to the sampling mask in a manner such that an outer contour of the organ structure of the investigated object is emphasized in the reconstructed magnetic resonance image data, wherein the identification of the organ structure of the investigated object is effected using the emphasized outer contour of the organ structure of the investigated object in the magnetic resonance image data.

In this manner, based on the organ structure already identified in the training magnetic resonance image data, it is possible to determine a specific pattern of how the organ structure to be identified or its outer contour appears in the k-space. This specific pattern, which can also be designated as the fingerprint or signature of the organ structure to be identified in the spatial frequency domain, can then offer the basis for definition of the sampling mask. In this manner, the sampling scheme can be tailored particularly suitably to identification of the organ structure.

The sampling mask can define a region in the k-space which comprises those relevant spatial frequencies that have to be sampled so that the outer contour of the organ structure of the investigated object is emphasized in the reconstructed magnetic resonance image data. To determine the sampling mask, a frequency analysis is carried out in particular of the outer contour of the organ structure of the at least one subject identified in the training magnetic resonance image data. The sampling mask can then be set as that relevant spatial frequency pattern in the k-space, the sampling of which results in representation of the outer contour of the organ structure in the magnetic resonance image data reconstructed from the magnetic resonance measurement data.

By way of example, a concrete procedure in this regard can be configured in this manner:
A plurality of training magnetic resonance image datasets which represent in each case the organ structure from various subjects is used as input parameters. With respect to the plurality of training magnetic resonance image datasets, the organ structure is already present and segmented or contoured in each case. Accordingly, the outer contour of the organ structure is present and extracted in each case in the plurality of training magnetic resonance image datasets.

An option for the further procedure is then to average across the plurality of outer contours of the organ structure in the plurality of training magnetic resonance image datasets, wherein an averaged outer contour of the organ structure is obtained. An inverse Fourier transform can then be carried out, resulting in obtaining that spatial frequency pattern, the sampling of which results in the averaged outer contour of the organ structure after reconstruction. The spatial frequency pattern can serve as the basis for defining the sampling mask.

Another option for the further procedure is to apply a Fourier transform to the extracted outer contour of the organ structure in the plurality of training magnetic resonance image datasets. A signature of the outer contour of the organ structure in the spatial frequency domain is therefore obtained for each training magnetic resonance image dataset. Relevant features of the signature of the outer contour of the organ structure in the spatial frequency domain can then be determined via a Principal Component Analysis (PCA). These can be used as the basis for creating the sampling mask for the concrete case.

An embodiment provides that the sampling scheme of the k-space supports the subsequent identification of the organ structure in the manner that the sampling scheme of the k-space results in a specific emphasizing or a specific saturation of the organ structure to be identified as compared with surrounding tissue in the magnetic resonance image data reconstructed from the magnetic resonance measurement data.

The sampling scheme can be selected in a manner such that the organ structure appears in the magnetic resonance image data with a particularly high signal as compared with the surrounding tissue or with the lowest possible signal as compared with the surrounding tissue or a zero signal. The employment of an inversion recovery sampling scheme, which selectively saturates the tissue of the organ structure as compared with the surrounding tissue, is conceivable in this regard, in a particularly advantageous manner. This technique can be employed advantageously in particular whenever the organ structure comprises fatty tissue, such as the liver for example. The specific emphasizing or specific saturation of the organ structure to be identified makes it possible to simplify identification of the organ structure.

An embodiment provides that the sampling scheme of the k-space supports the subsequent identification of the organ structure in the manner that the sampling scheme of the k-space results in a measurement geometry specifically coordinated to the organ to be identified in the magnetic resonance image data reconstructed from the magnetic resonance measurement data.

The measurement geometry can be advantageously coordinated to the organ structure to be identified in the manner that the sampling scheme exclusively samples specific layers that are particularly suitable for identification of the organ structure or the outer contour of the organ structure. It is also possible to sample a plurality of layers that intersect in a region of the organ structure and therefore can provide redundant information along various layer directions in the region of the organ structure. Employment of a so-called zoomed imaging method is also conceivable, in which the sampling scheme provides a measurement field (field of view, FOV) which is reduced to the region of the organ structure. It can also be beneficial to select a measurement geometry in which changes in the organ structure over time, for example due to a breathing movement of the investigated objects, can be detected particularly advantageously.

An embodiment provides that the identification of the organ structure comprises a segmentation and/or contouring of the organ structure for employment in the course of a radiation treatment of the investigated object.

In particular, the segmented and/or contoured organ structure can be employed as the target organ or risk organ in planning the radiation treatment of the investigated object. Furthermore, it is possible to employ the segmented and/or contoured organ structure when setting up the patient for treatment in the treatment room, e.g. by monitoring the correct positioning of the target organ. A further application is the monitoring of the target organ and/or risk organs during the application of the radiation treatment beam. In the latter case the magnetic resonance machine advantageously is a part of a combined MR-LINAC machine. The magnetic resonance machine can also be positioned near the treatment room, in which the LINAC machine is installed, whereas advantageously a patient transfer is organized between the magnetic resonance machine and the LINAC machine. In this manner, the proposed procedure enables acquisition of magnetic resonance image data which is particularly suitable for employment in the course of the radiation treatment of the investigated object.

Furthermore, a possible application of the segmented and/or contoured organ structure is the planning and/or preparation and/or monitoring of a MR-guided interventional procedure regarding the organ structure of the patient. The identification of the organ structure can also be used intraoperatively.

An embodiment provides that a further acquisition of further magnetic resonance measurement data of the organ structure of the investigated object is effected by way of the magnetic resonance machine using a further magnetic resonance sequence, wherein further magnetic resonance image data is reconstructed from the further magnetic resonance measurement data, wherein the organ structure segmented and/or contoured on the basis of the magnetic resonance image data is compared with an appearance of the organ structure in the further magnetic resonance image data for the purpose of quality assurance.

The further magnetic resonance measurement data can be acquired at an earlier time or at a later time than the magnetic resonance measurement data. The organ structure segmented and/or contoured on the basis of the magnetic resonance image data can be presented merged with the further magnetic resonance image data for the comparison. It is also possible to compare parameters, such as a volume for example, of the organ structure segmented and/or contoured on the basis of the magnetic resonance image data with the same organ structure segmented and/or contoured on the basis of the further magnetic resonance image data. Naturally, a person skilled in the art is familiar with further options for comparison.

With the aid of this proposed procedure, it is advantageously possible to ensure an adequate quality of the magnetic resonance measurement data acquired by way of the sampling scheme for employment in the course of a treatment of the patient. By way of advantage, a visual quality assurance is effected in this regard with respect to the further magnetic resonance image data which has been acquired in particular by way of a conventional sampling scheme. The quality assurance can ensure for example that the spatial geometry of the organ structure was not distorted in any relevant manner by the sampling scheme used. Quality assurance of the organ structure segmented and/or contoured on the basis of the magnetic resonance image data is also conceivable with respect to an atlas containing a typical appearance of the organ structure for a population.

The inventive magnetic resonance machine comprises a measurement data acquisition unit and an arithmetic unit, wherein the magnetic resonance machine is realized for implementing an inventive method.

In particular, the measurement data acquisition unit is realized for acquiring magnetic resonance measurement data of the organ structure of the investigated object using a magnetic resonance sequence which specifies a sampling scheme of a k-space. In this respect, the sampling scheme of the k-space supports the subsequent identification of the organ structure in the magnetic resonance image data reconstructed from the magnetic resonance measurement data.

At the same time, the arithmetic unit can be realized so as to send control signals to the magnetic resonance machine and/or receive control signals and/or process them, in order to implement at least part of the inventive method. The arithmetic unit can be integrated in to the magnetic resonance machine. The arithmetic unit can also be installed separately from the magnetic resonance machine. The arithmetic unit can be connected to the magnetic resonance machine. In particular, in order to implement at least part of the inventive method, the arithmetic unit has a reconstruction unit for reconstructing magnetic resonance image data from the magnetic resonance measurement data and an identification unit for identifying the organ structure in the magnetic resonance image data.

The components of the arithmetic unit of the inventive magnetic resonance machine such as the reconstruction unit or the identification unit can be realized for the most part in the form of software components. Fundamentally, however, these components can also be partly realized, especially where particularly rapid calculations are involved, in the form of software-supported hardware components such as FPGAs or similar. The necessary interfaces can likewise be realized as software interfaces, for example when just importing data from other software components is involved. But they can also be realized in the form of hardware-based interfaces that are activated by suitable software. It is naturally also conceivable for a plurality of the said components to be realized in combination in the form of a single software component or software-supported hardware component.

The computer program product according to the invention can be loaded directly into a memory of a programmable computing unit of a magnetic resonance machine and has program-code means for carrying out a method according to the invention when the computer program product is executed in the computing unit of the magnetic resonance machine. This enables the method according to the invention to be carried out quickly, identically repeatedly and robustly. The computer program product is configured such that it can carry out the method steps according to the invention by means of the computing unit. Here, the computing unit must in each case meet the requirements for carrying out the respective method steps efficiently, for example have a corresponding working memory, a corresponding graphics card or a corresponding logic board. The computer program product is, for example, stored on a computer-readable medium or on a network or server from where it can be loaded into the processor of a local computing unit, which can be directly connected to the magnetic resonance machine or embodied as part of the magnetic resonance machine. Furthermore, control information for the computer program product can be stored on an electronically readable data carrier. The control information on the electronically readable data carrier can be embodied such that, when the data carrier is used in a computing unit of the magnetic resonance machine, it carries out a method according to the invention. Examples of electronically readable data carrier are a DVD, a magnetic tape or a USB stick on which electronically readable control information, in particular software (see above), is stored. If this control information (software) is read by the data carrier and stored in a control system and/or computing unit of the magnetic resonance machine, it is possible to carry out all embodiments of the invention of the above-described method. In this way, the invention can also be based on said computer-readable medium and/or said electronically readable data carrier.

The advantages of the magnetic resonance machine according to the invention and the computer program product according to the invention substantially correspond to the advantages of the method according to the invention, which were explained in detail above. Features, advantages or alternative embodiments mentioned here should also be transferred to the other claimed objects and vice versa. In other words, the objective claims can also be developed with the features described or claimed in connection with a method. The corresponding functional features of the method are achieved by corresponding objective modules, in particular by hardware modules.

The inventive magnetic resonance machine 11 comprises a measurement data acquisition unit 32. The arithmetic unit 24 has a reconstruction unit 33 for reconstructing magnetic resonance image data from the magnetic resonance measurement data and an identification unit 34 for identifying the organ structure in the magnetic resonance image data.

The invention will be described and illustrated in more detail below with reference to the exemplary embodiments illustrated in the figures, in which:
- Fig. 1: shows an inventive magnetic resonance machine in a schematic view,
- Fig. 2: shows a flowchart of a first embodiment of an inventive method,
- Fig. 3: shows a flowchart of a second embodiment of an inventive method,
- Fig. 4: an exemplary sampling radial scheme which supports the subsequent identification of the organ structure and
- Fig. 5: an exemplary sampling radial scheme which supports the subsequent identification of the organ structure.

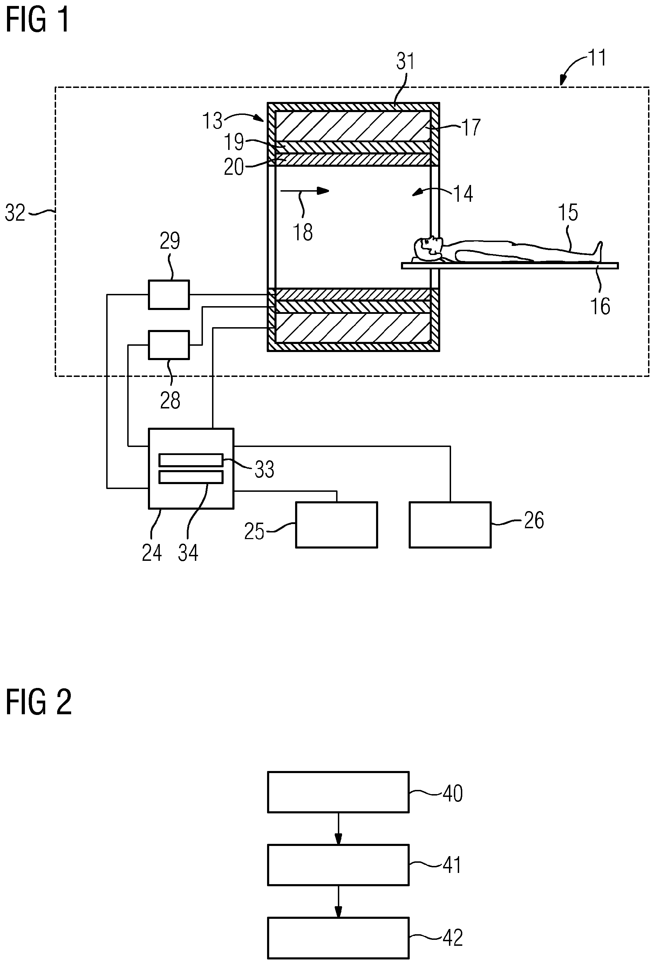

**Fig. 1** schematically illustrates an inventive magnetic resonance machine 11. The magnetic resonance machine 11 comprises a detector unit formed by a magnetic unit 13 and having a main magnet 17 for generating a strong and, in particular, constant main magnetic field 18. In addition, the magnetic resonance machine 11 has a cylindrical patient-receiving region 14 for receiving an examination object 15, in the present case a patient, with the patient-receiving region 14 being cylindrically surrounded in a circumferential direction by the magnetic unit 13. The patient 15 can be pushed by means of a patient-positioning device 16 of the magnetic resonance machine 11 into the patient-receiving region 14. The patient-positioning device 16 has for this purpose an examination table arranged so as to move inside the magnetic resonance machine 11. The magnetic unit 13 is shielded from the outside by means of a housing panel 31 of the magnetic resonance machine 11.

The magnetic unit 13 also has a gradient coil unit 1 for generating magnetic field gradients which are used for spatial encoding during imaging. The gradient coil unit 19 is controlled by means of a gradient control unit 28. The magnetic unit 13 also comprises a high-frequency antenna unit 20, which in the illustrated case is designed as a body coil permanently integrated in the magnetic resonance machine 11, and a high-frequency antenna control unit 29 for exciting a polarization that is established in the main magnetic field 18 generated by the main magnet 17. The high-frequency antenna unit 20 is controlled by a high-frequency antenna control unit 29 and irradiates high-frequency magnetic resonance sequences into an examination room which is formed substantially by the patient-receiving region 14. The high-frequency antenna unit 20 is also designed to receive magnetic resonance signals, in particular from the patient 15.

For controlling the main magnet 17, the gradient control unit 28 and the high-frequency antenna control unit 29, the magnetic resonance machine 11 has an arithmetic unit 24. The arithmetic unit 24 centrally controls the magnetic resonance machine 11, such as for example carrying out a predetermined imaging gradient echo sequence. Control information such as imaging parameters, and reconstructed magnetic resonance images can be supplied on a supply unit 25, in the present case a display unit 25, of the magnetic resonance machine 11 for a user. In addition, the magnetic resonance machine 11 has an input unit 26, by means of which information and/or parameters can be input by a user during a scanning process. The arithmetic unit 24 can comprise the gradient control unit 28 and/or high-frequency antenna control unit 29 and/or the display unit 25 and/or the input unit 26. The arithmetic unit 24 has a reconstruction unit 33 for reconstructing magnetic resonance image data from the magnetic resonance measurement data and an identification unit 34 for identifying the organ structure in the magnetic resonance image data.

Thus the magnetic resonance machine 11 with the measurement data acquisition unit 32 and the arithmetic unit 24 is embodied to carry out the inventive method (see following Figures).

The magnetic resonance machine 11 shown can obviously include further components normally comprised by magnetic resonance machines 11. Moreover, the general mode of operation of a magnetic resonance machine 11 is known to the person skilled in the art so there will be no detailed description of the further components.

Fig. 2 shows a flowchart of a first embodiment of an inventive method.

In a first method step 40 the measurement data acquisition unit 32 of the magnetic resonance machine 11 acquires the magnetic resonance measurement data for the organ structure of the investigated object using a magnetic resonance sequence which specifies a sampling scheme of a k-space.

In a further method step 41 the reconstruction unit 33 of the arithmetic unit 24 reconstructs the magnetic resonance image data from the magnetic resonance measurement data.

In a further method step 42 the identification unit 34 of the arithmetic unit 24 identifies of the organ structure in the magnetic resonance image data.

The sampling scheme of the k-space supports the subsequent identification of the organ structure in the magnetic resonance image data reconstructed from the magnetic resonance measurement data.

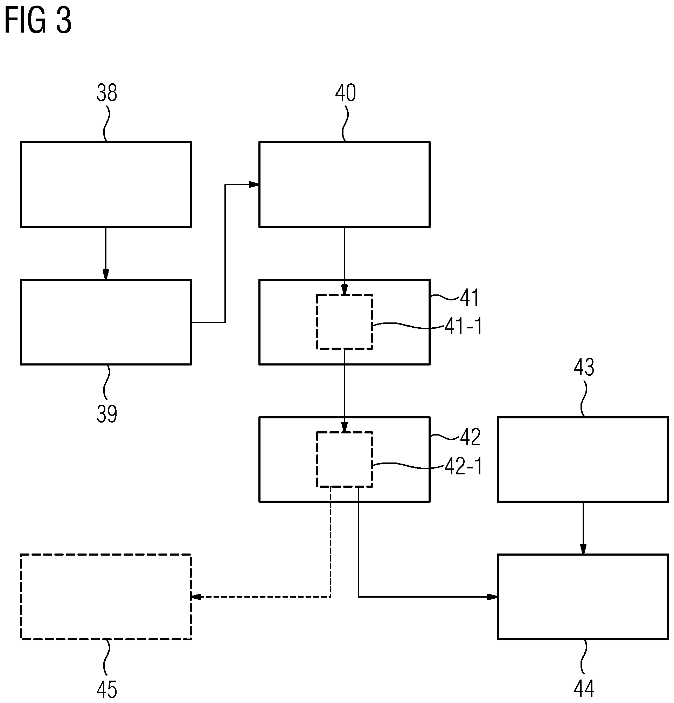

Fig. 3 shows a flowchart of a second embodiment of an inventive method.

The following description is substantially limited to the differences from the exemplary embodiment in Fig. 2, with reference being made to the description of the exemplary embodiment in Fig. 2 in relation to identical method steps. Method steps that are substantially the same are basically numbered with the same reference numerals.

The embodiment of the inventive method shown in Fig. 3 substantially comprises the method steps 40, 41, 42 of the first embodiment of the inventive method according to Fig. 2. In addition, the embodiment of the inventive method shown in Fig. 3 comprises additional method steps and/or substeps. An alternative procedure to that in Fig. 3, which has only some of the additional method steps and/or substeps shown in Fig. 3, is also conceivable. Of course an alternative procedure to that in Fig. 3 can also have additional method steps and/or substeps.

There are different possibilities how the sampling scheme of the k-space can support the subsequent identification of the organ structure. Figs. 3-5 show that the sampling scheme of the k-space supports the subsequent identification of the organ structure in the manner that the sampling scheme of the k-space provides for a higher sampling density in an outer region of the k-space than in a central region of the k-space. The higher sampling density in the outer region than in the central region results in an emphasizing of an outer contour of the organ structure in the reconstructed magnetic resonance image data in a substep 41-1 of the method step 41. The identification of the organ structure is effected using the emphasized outer contour of the organ structure in the magnetic resonance image data in a substep 42-2 of the method step 42.

In a further method step 38 training magnetic resonance image data are acquired from at least one subject other than the investigated object. The organ structure of the at least one subject is already present and identified in the training magnetic resonance image data. In a further method step 39 the sampling scheme of the k-space is determined based on training magnetic resonance image data. This comprises a calculation of a sampling mask based on an outer contour of the organ structure of the at least one subject identified in the training magnetic resonance image data, wherein the sampling scheme of the k-space provides for a sampling of the k-space according to the sampling mask in a manner such that an outer contour of the organ structure of the investigated object is emphasized in the reconstructed magnetic resonance image data.

The identification of the organ structure comprises a segmentation and/or contouring of the organ structure in the substep 42-1 of the further method step 42. This segmented and/or contoured organ structure can be employed in the planning of a radiation treatment of the investigated object in a further method step 45.

Additionally, a further acquisition of further magnetic resonance measurement data of the organ structure of the investigated object can be effected by way of the magnetic resonance machine using a further magnetic resonance sequence in a further method step 43, wherein further magnetic resonance image data is reconstructed from the further magnetic resonance measurement data. In a further method step 44, the organ structure segmented and/or contoured on the basis of the magnetic resonance image data can be compared with an appearance of the organ structure in the further magnetic resonance image data for the purpose of quality assurance.

One other possibility how the sampling scheme of the k-space can support the subsequent identification of the organ structure is that the sampling scheme of the k-space results in a specific emphasizing or a specific saturation of the organ structure to be identified as compared with surrounding tissue in the magnetic resonance image data reconstructed from the magnetic resonance measurement data.

One other possibility how the sampling scheme of the k-space can support the subsequent identification of the organ structure is that the sampling scheme of the k-space results in a measurement geometry specifically coordinated to the organ to be identified in the magnetic resonance image data reconstructed from the magnetic resonance measurement data.

The method steps of the method according to the invention shown in Figs. 2-3 are carried out by the computing unit. To this end, the computing unit comprises the necessary software and/or computer programs, which are stored in a memory unit of the computing unit. The software and/or computer programs comprise program means designed to carry out the method according to the invention when the computer program and/or software is executed in the computing unit by means of a processor unit of the computing unit.

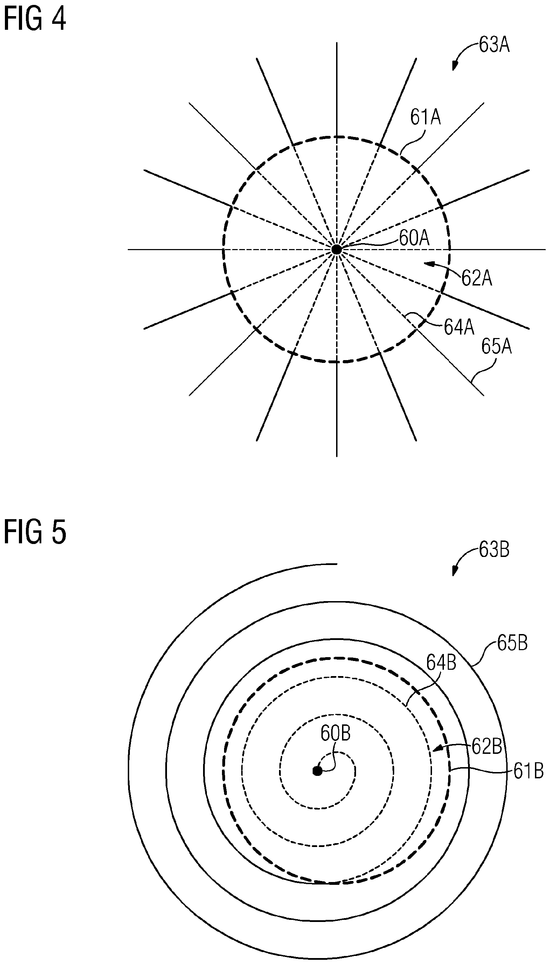

**Figs. 4 and 5** both show exemplary sampling schemes which can support the subsequent identification of the organ structure. Both Figures only show schematic representations of the sampling schemes which only serve an illustrative purpose. Of course, other sampling schemes are possible, e.g. cartesian sampling schemes or other forms of sub-sampling.

In both Figures the sampling path in the k-space is depicted. The k-space is divided into a central region 62A, 62B and an outer region 63A, 63B. The central region 62A, 62B surrounds the center 60A, 60B of k-space. The outer region 63A, 63B surrounds the central region 62A, 62B. The central region 62A, 62B is delimited from the outer region 63A, 63B by a boundary line 61A, 61B. In Figs. 4-5 the boundary line 61, 61B is a circle. However, any suitable shape can be chosen for the boundary line 61A, 61B between the outer region 63A, 63B and the central region 62A, 62B.

The sampling scheme of the k-space provides for a higher sampling density in the outer region 63A, 63B of the k-space than in a central region 62A, 62B of the k-space. Particularly, the sampling scheme of the k-space provides for a sampling of the k-space exclusively in the outer region 63A, 63B. Thereby, Fig. 4 shows a radial sampling of the k-space exclusively in the outer region 63A. Fig. 5 shows a spiral sampling of the k-space exclusively in the outer region 63B.

In the radial sampling shown in Fig. 4 the k-space is sampled in several spokes which pass through the center 60A of k-space. A spoke is split into a central part 64A and an outer part 65A. The central part 64A of the spoke lies in the central region 62A of the k-space. The outer part 65A of the spoke is positioned in the outer region 63A of the k-space. Only the outer part 65A of the spoke is sampled according to the sampling scheme of Fig. 4.

In the spiral sampling shown in Fig. 5 the k-space is sampled according to a spiral which originates in the center 60B of k-space. The spiral is split into a central part 64B and an outer part 65B. The central part 64B of the spiral lies in the central region 62B of the k-space. The outer part 65B of the spiral is positioned in the outer region 63B of the k-space.

Only the outer part 65B of the spiral is sampled according to the sampling scheme of Fig. 5.

Although the invention has been illustrated and described in more detail by the preferred exemplary embodiment, it is not limited by the disclosed examples and a person skilled in the art can derive other variations herefrom without departing from the scope of the invention.

## Claims

1. A method for identifying an organ structure of an investigated object in magnetic resonance image data, comprising the following method steps:
- acquisition of magnetic resonance measurement data for the organ structure of the investigated object by way of a magnetic resonance machine using a magnetic resonance sequence which specifies a sampling scheme of a k-space,
- reconstruction of magnetic resonance image data from the magnetic resonance measurement data, and
- identification of the organ structure in the magnetic resonance image data, wherein the identification of the organ structure in the magnetic resonance image data comprises a segmentation of the organ structure by way of a segmentation algorithm and/or a contouring of the organ structure,
wherein the sampling scheme of the k-space supports the subsequent identification of the organ structure in the magnetic resonance image data reconstructed from the magnetic resonance measurement data,
**characterized in that** the sampling scheme of the k-space supports the subsequent identification of the organ structure in the manner that the sampling scheme of the k-space provides for a higher sampling density in an outer region of the k-space than in a central region of the k-space,
wherein the higher sampling density in the outer region than in the central region results in an emphasizing of an outer contour of the organ structure in the reconstructed magnetic resonance image data,
wherein the identification of the organ structure is effected using the emphasized outer contour of the organ structure in the magnetic resonance image data.

2. The method as claimed in claim 1, wherein the sampling scheme of the k-space provides for a sampling of the k-space exclusively in the outer region.

3. The method as claimed in one of the preceding claims, wherein the sampling scheme of the k-spaces provides for a spiral or radial sampling of the k-space with a higher sampling density in the outer region than in the central region.

4. The method as claimed in one of the preceding claims, wherein the sampling scheme of the k-space is determined based on training magnetic resonance image data, wherein the training magnetic resonance image data has been acquired from at least one subject other than the investigated object, and the organ structure of the at least one subject is already present and identified in the training magnetic resonance image data, wherein the determining of the sampling scheme of the k-space based on the training magnetic resonance image data comprises a calculation of a sampling mask based on an outer contour of the organ structure of the at least one subject identified in the training magnetic resonance image data, wherein the sampling scheme of the k-space provides for a sampling of the k-space according to the sampling mask in a manner such that an outer contour of the organ structure of the investigated object is emphasized in the reconstructed magnetic resonance image data, wherein the identification of the organ structure of the investigated object is effected using the emphasized outer contour of the organ structure of the investigated object in the magnetic resonance image data.

5. The method as claimed in one of the preceding claims, wherein the sampling scheme of the k-space supports the subsequent identification of the organ structure in the manner that the sampling scheme of the k-space results in a specific emphasizing or a specific saturation of the organ structure to be identified as compared with surrounding tissue in the magnetic resonance image data reconstructed from the magnetic resonance measurement data.

6. The method as claimed in one of the preceding claims, wherein the sampling scheme of the k-space supports the subsequent identification of the organ structure in the manner that the sampling scheme of the k-space results in a measurement geometry specifically coordinated to the organ to be identified in the magnetic resonance image data reconstructed from the magnetic resonance measurement data.

7. The method as claimed in one of the preceding claims, wherein the segmentation and/or contouring of the organ structure is employed in the course of a radiation treatment of the investigated object.

8. The method as claimed in claim 7, wherein a further acquisition of further magnetic resonance measurement data of the organ structure of the investigated object is effected by way of the magnetic resonance machine using a further magnetic resonance sequence, wherein further magnetic resonance image data is reconstructed from the further magnetic resonance measurement data, wherein the organ structure segmented and/or contoured on the basis of the magnetic resonance image data is compared with an appearance of the organ structure in the further magnetic resonance image data for the purpose of quality assurance.

9. A magnetic resonance machine, comprising a measurement data acquisition unit and an arithmetic unit, wherein the magnetic resonance machine is configured to implement a method as claimed in one of the preceding claims.

10. A computer program product, which can be loaded directly in to a memory of a programmable arithmetic unit of a magnetic resonance machine, together with program code means to implement a method as claimed in one of claims 1-8 when the computer program product is executed in the arithmetic unit of the magnetic resonance machine.

## Patentansprüche

1. Verfahren zum Identifizieren einer Organstruktur eines untersuchten Objekts in Magnetresonanz-Bilddaten, das die folgenden Verfahrensschritte umfasst:
- Erfassen von Magnetresonanz-Messdaten für die Organstruktur des untersuchten Objekts durch eine Magnetresonanzmaschine unter Verwendung einer Magnetresonanzsequenz, die ein Abtastschema eines k-Raums spezifiziert,
- Rekonstruieren von Magnetresonanz-Bilddaten aus den Magnetresonanz-Messdaten und
- Identifizieren der Organstruktur in den Magnetresonanz-Bilddaten, wobei das Identifizieren der Organstruktur in den Magnetresonanz-Bilddaten ein Segmentieren der Organstruktur durch einen Segmentierungsalgorithmus und/oder ein Konturieren der Organstruktur umfasst,
wobei das Abtastschema des k-Raums das anschließende Identifizieren der Organstruktur in den Magnetresonanz-Bilddaten, die aus den Magnetresonanz-Messdaten rekonstruiert worden sind, unterstützt,
**dadurch gekennzeichnet, dass** das Abtastschema des k-Raums das anschließende Identifizieren der Organstruktur in den Magnetresonanz-Bilddaten derart unterstützt, dass das Abtastschema des k-Raums in einem äußeren Bereich des k-Raums für eine höhere Abtastdichte sorgt als in einem zentralen Bereich des k-Raums,
wobei die höhere Abtastdichte in dem äußeren Bereich als in dem zentralen Bereich in einem Hervorheben einer äußeren Kontur der Organstruktur in den rekonstruierten Magnetresonanz-Bilddaten resultiert,
wobei das Identifizieren der Organstruktur unter Verwendung der hervorgehobenen äußeren Kontur der Organstruktur in den Magnetresonanz-Bilddaten bewirkt wird.

2. Verfahren nach Anspruch 1, wobei das Abtastschema des k-Raums für ein Abtasten des k-Raums ausschließlich in dem äußeren Bereich sorgt.

3. Verfahren nach einem der vorhergehenden Ansprüche, wobei das Abtastschema des k-Raums für ein spiralförmiges oder radiales Abtasten des k-Raums mit einer höheren Abtastdichte in dem äußeren Bereich als in dem zentralen Bereich sorgt.

4. Verfahren nach einem der vorhergehenden Ansprüche, wobei das Abtastschema des k-Raums basierend auf Trainingsmagnetresonanz-Bilddaten bestimmt wird, wobei die Trainingsmagnetresonanz-Bilddaten von mindestens einem anderen Versuchsobjekt als dem untersuchten Objekt erfasst worden sind, und die Organstruktur des mindestens einen Versuchsobjekts bereits in den Trainingsmagnetresonanz-Bilddaten vorhanden und identifiziert sind, wobei das Bestimmen des Abtastschemas des k-Raums basierend auf den Trainingsmagnetresonanz-Bilddaten ein Berechnen einer Abtastmaske basierend auf einer äußeren Kontur der Organstruktur des mindestens einen Versuchsobjekts, die in den Trainingsmagnetresonanz-Bilddaten identifiziert worden ist, umfasst, wobei das Abtastschema des k-Raums für ein Abtasten des k-Raums entsprechend der Abtastmaske derart sorgt, dass eine äußere Kontur der Organstruktur des untersuchten Objekts in den rekonstruierten Magnetresonanz-Bilddaten hervorgehoben wird, wobei das Identifizieren der Organstruktur des untersuchten Objekts unter Verwendung der hervorgehobenen äußeren Kontur der Organstruktur des untersuchten Objekts in den Magnetresonanz-Bilddaten bewirkt wird.

5. Verfahren nach einem der vorhergehenden Ansprüche, wobei das Abtastschema des k-Raums das nachfolgende Identifizieren der Organstruktur derart unterstützt, dass das Abtastschema des k-Raums in einer spezifischen Hervorhebung oder einer spezifischen Sättigung der Organstruktur, die identifiziert werden soll, im Vergleich mit dem umgebenden Gewebe in den Magnetresonanz-Bilddaten, die aus den Magnetresonanz-Messdaten rekonstruiert worden sind, resultiert.

6. Verfahren nach einem der vorhergehenden Ansprüche, wobei das Abtastschema des k-Raums das nachfolgende Identifizieren der Organstruktur derart unterstützt, dass das Abtastschema des k-Raums in einer Messgeometrie, die speziell auf das Organ abgestimmt ist, das in den Magnetresonanz-Bilddaten identifiziert werden soll, die aus den Magnetresonanz-Messdaten rekonstruiert worden sind, resultiert.

7. Verfahren nach einem der vorhergehenden Ansprüche, wobei das Segmentieren und/oder das Konturieren der Organstruktur im Verlauf einer Strahlenbehandlung des untersuchten Objekts eingesetzt wird.

8. Verfahren nach Anspruch 7, wobei eine weitere Erfassung weiterer Magnetresonanz-Messdaten der Organstruktur des untersuchten Objekts durch die Magnetresonanz-Maschine unter Verwendung einer weiteren Magnetresonanzsequenz bewirkt wird, wobei weitere Magnetresonanz-Bilddaten aus den weiteren Magnetresonanz-Messdaten rekonstruiert werden, wobei die Organstruktur, die auf der Grundlage der Magnetresonanz-Bilddaten segmentiert und/oder konturiert worden sind, zum Zweck der Qualitätssicherung mit einem Erscheinungsbild der Organstruktur in den weiteren Magnetresonanz-Bilddaten verglichen wird.

9. Magnetresonanzmaschine, die eine Messdaten-Erfassungseinheit und eine Arithmetikeinheit umfasst, wobei die Magnetresonanzmaschine konfiguriert ist, ein Verfahren nach einem der vorhergehenden Ansprüche zu implementieren.

10. Computerprogrammprodukt, das zusammen mit Programmcode-Mitteln direkt in einen Speicher einer programmierbaren Arithmetikeinheit einer Magnetresonanzmaschine geladen werden kann, um ein Verfahren nach einem der Ansprüche 1-8 zu implementieren, wenn das Computerprogrammprodukt in der Arithmetikeinheit der Magnetresonanzmaschine ausgeführt wird.

## Revendications

1. Procédé d'identification de la structure d'organe d'un objet à examiner dans des données d'image par résonance magnétique, comprenant les stades de procédé suivant :
- acquisition de données de mesure par résonance magnétique de la structure d'organe de l'objet à examiner au moyen d'une machine à résonance magnétique utilisant une séquence de résonance magnétique, qui spécifie un schéma d'échantillonnage d'un espace k,
- reconstruction de données d'image par résonance magnétique à partir des données de mesure par résonance magnétique, et
- identification de la structure d'organe dans des données d'image par résonance magnétique, dans lequel l'identification de la structure d'organe dans les données d'image par résonance magnétique comprend une segmentation de la structure d'organeau moyen d'un algorithme de segmentation et/ou un contourage de la structure de l'organe, dans lequel le schéma d'échantillonnage de l'espace k supporte l'identification subséquente de la structure d'organe dans les données d'image par résonance magnétique reconstruites à partir des données de mesure par résonance magnétique,
**caractérisé en ce que** le schéma d'échantillonnage de l'espace k supporte l'identification subséquente de la structure de l'organe, en ce sens que le schéma d'échantillonnage de l'espace k donne une densité d'échantillonnage plus grande dans une région extérieure de l'espace k que dans une région centrale de l'espace k,
dans lequel la densité d'échantillonnage plus grande dans la région extérieure que dans la région centrale se traduit par une accentuation d'un contour extérieur de la structure d'organe dans les données d'image par résonance magnétique reconstruites,
dans lequel l'identification de la structure d'organe est effectuée en utilisant le contour extérieur accentué de la structure d'organe dans les données d'image par résonance magnétique.

2. Procédé suivant la revendication 1, dans lequel le schéma d'échantillonnage de l'espace k donne un échantillonnage de l'espace k exclusivement dans la région extérieure.

3. Procédé suivant l'une quelconque des revendications précédentes, dans lequel le schéma d'échantillonnage de l'espace K donne un échantillonnage en spirale ou radiale de l'espace k ayant une densité d'échantillonnage plus grande dans la région extérieure que dans la région centrale.

4. Procédé suivant l'une quelconque des revendications précédentes, dans lequel on détermine le schéma d'échantillonnage de l'espace k sur la base de données d'image par résonnance magnétique d'apprentissage, dans lequel les données d'image par résonance magnétique d'apprentissage ont été acquises chez au moins un sujet autre que l'objet examiné et la structure d'organe du au moins un sujet est déjà présente et identifiée dans les données d'image par résonnance magnétique d'apprentissage, dans lequel la détermination du schéma d'échantillonnage de l'espace k sur la base des données d'image par résonance magnétique d'apprentissage comprend un calcul d'un masque d'échantillonnage sur la base d'un contour extérieur de la structure d'organe du au moins sujet identifié dans les données d'image par résonance magnétique d'apprentissage, dans lequel le schéma d'échantillonnage de l'espace k donne un échantillonnage de l'espace k suivant le masque d'échantillonnage en ce sens qu'un contour extérieur de la structure d'organe de l'objet examiné est accentué dans les données d'image par résonance magnétique reconstruites, dans lequel l'identification de la structure d'organe de l'objet examiné est effectuée en utilisant le contour extérieur accentuée de la structure d'organe de l'objet examiné dans les données d'image par résonance magnétique.

5. Procédé suivant l'une quelconque des revendications précédentes, dans lequel le schéma d'échantillonnage de l'espace k supporte l'identification subséquente de la structure d'organe en ce sens que le schéma d'échantillonnage de l'espace k se traduit par une accentuation spécifique ou par une saturation spécifique de la structure d'organe à identifier par rapport à des tissus environnants dans les données d'image par résonance magnétique reconstruites à partir des données de mesure par résonance magnétique

6. Procédé suivant l'une quelconque des revendications précédentes, dans lequel le schéma d'échantillonnage de l'espace k supporte l'identification subséquente de la structure d'organe en ce sens que le schéma d'échantillonnage de l'espace k se traduit par une géométrie de mesure coordonnée spécifiquement avec l'organe à identifier dans les données d'image par résonance magnétique reconstruites à partir des données de mesure par résonance magnétique.

7. Procédé suivant l'une quelconque des revendications précédentes, dans lequel la segmentation et/ou le contourage de la structure d'organe est employé au cours d'un traitement par rayonnement de l'objet à examiner.

8. Procédé suivant la revendication 7, dans lequel on effectue une autre acquisition d'autres données de mesure par résonance magnétique de la structure d'organe de l'objet à examiner au moyen de la machine à résonance magnétique, en utilisant une autre séquence de résonance magnétique, dans lequel on reconstruit d'autres données d'image par résonance magnétique à partir des autres données de mesure de résonance magnétique, dans lequel on compare la structure d'organe segmenté et/ou conturée sur la base des données d'image par résonance magnétique à un aspect de la structure d'organe dans les autres données d'image par résonance magnétique afin de s'assurer de la qualité.

9. Machine à résonance magnétique, comprenant une unité d'acquisition de données de mesure et une unité arithmétique, dans laquelle la machine à résonance magnétique est configurée pour mettre en œuvre un procédé tel que revendiqué dans l'une quelconque des revendications précédentes.

10. Produit de programme d'ordinateur, qui peut être chargé directement dans une mémoire d'une unité arithmétique programmable d'une machine à résonance magnétique ensemble avec des moyens de code de programme pour mettre en œuvre un procédé tel que revendiqué dans l'une quelconque des revendications 1 à 8, lorsque le produit de programme d'ordinateur est exécuté dans l'unité arithmétique de la machine à résonance magnétique.
